# EUROPEAN PATENT APPLICATION

(11) **EP 1 997 878 A1**
(43) Date of publication of application: **03.12.2008**
(21) Application number: 08157325.5
(22) Date of filing: 30.05.2008
(51) Int. Cl.: C12M 3/00

(54) **CELL CAPTURING PLATE, MICROINJECTION APPARATUS, AND METHOD OF PRODUCING CELL CAPTURING PLATE**

(30) Priority: 31.05.2007 JP 2007145409
(71) Applicant: Fujitsu Limited, Kawasaki-shi, Kanagawa 211-8588 (JP)
(72) Inventor: ITO,, Akio, Kawasaki-shi, Kanagawa 211-8588 (JP); YABUKI,, Akihiko, Kawasaki-shi, Kanagawa 211-8588 (JP); UCHIDA,, Daisuke, Kawasaki-shi, Kanagawa 211-8588 (JP); SAKAI,, Satoru, Kawasaki-shi, Kanagawa 211-8588 (JP); YOUOKU,, Sachihiro, Kawasaki-shi, Kanagawa 211-8588 (JP)
(74) Representative: Stebbing, Timothy Charles

(57) **Abstract**

A cell capturing plate (103) has a flat surface (202), an opening formed in one surface of the flat surface (202) and supporting the cell therein, and a through-hole penetrating from the opening to the other surface of the flat surface and having a cross section parallel to the flat surface (202) smaller than that of the opening.

## Description

This application claims the benefit of Application No. 2007-145409, filed May 31, 2007, in the Japan Intellectual Property Office.

The present invention relates to a cell capturing plate having a flat surface for capturing a cell, i.e., an injection target, to which a material is injected through a capillary needle capable of being projected along its longitudinal axis, a microinjection apparatus, and a method of producing the cell capturing plate. More particularly, the present invention relates to a cell capturing plate, a microinjection apparatus, and a method of producing the cell capturing plate, which can capture cells having different diameters and can reliably inject medical fluid into each of the captured cells.

Recently, efforts have been focused on research for directly injecting genes, antibodies, proteins, etc. into cells, to thereby alter the genetic information of the cells and/or to analyze changes appearing in the cells. The progress of this research contributes not only to clarifying the roles of the genes and the functions of biomolecules such as antibodies and proteins, but also realizing, for example, tailor-made medical care, i.e., genetic care matched with personal genetic characteristics. As examples of a method for injecting genes into cells, there are proposed an electrical method (electroporation), a chemical method (lipofection), a biological method (vector method), a mechanical method (microinjection), and an optical method (laser injection). However, the electrical method is disadvantageous in causing substantial damage of the cell because a cell membrane is broken due to application of a large current. The chemical and biological methods are disadvantageous in having lower efficiency because materials capable of being introduced by these methods are limited. The biological method is further disadvantageous in that it carries a risk of infection, etc.

On the other hand, the mechanical method, i.e., the microinjection is considered safest and a highly-efficient method. The microinjection is a technique useful for injecting, in addition to genes, biomolecules such as antibodies and proteins, and chemical compounds (hereinafter referred to collectively as "medical fluids") into cells.

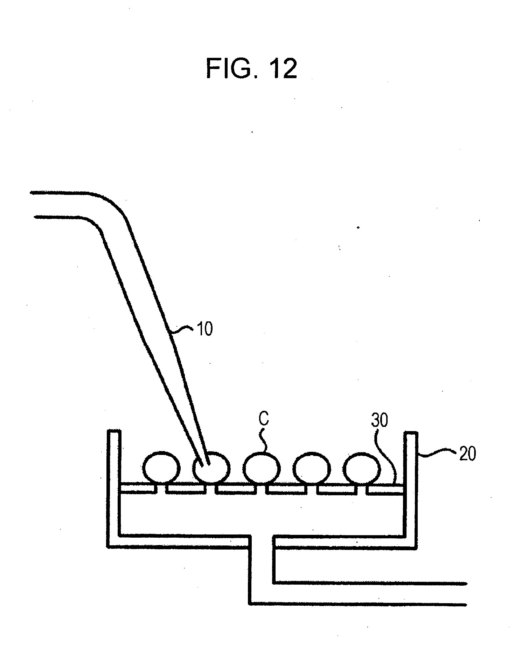

In the microinjection, as shown in Fig. 12, a capillary needle 10 is automatically moved to a position of a cell C and a medical fluid is directly injected into the cell C. At that time, the position of the cell C in a petri dish 20 has to be fixedly held in order to ensure that the capillary needle 10 is accurately moved to the position of the cell C. To that end, in Fig. 12, a cell capturing plate 30 formed with a plurality of through-holes is placed in the petri dish 20 and the underside of the cell capturing plate 30 facing the bottom of the petri dish 20 is subjected to negative pressure so that the cell C is sucked (attracted) to the through-hole in the cell capturing plate 30.

More specifically, in Fig. 12, a buffer fills the petri dish 20, and the cell capturing plate 30 and the cells C are immersed in the buffer. The buffer is sucked through the bottom surface of the petri dish 20 to generate negative pressure at the underside of the cell capturing plate 30 facing the bottom of the petri dish 20, whereby the cells C are sucked to the through-holes bored in the cell capturing plate 30. Thus, the positions of the cells C are fixed to the positions of the through-holes in the cell capturing plate 30. Accordingly, if the coordinates of each through-hole are stored in advance, the capillary needle 10 can be accurately moved to the position of the cell C.

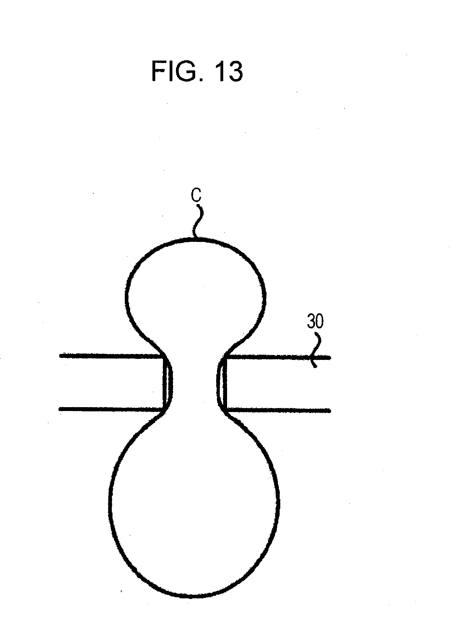

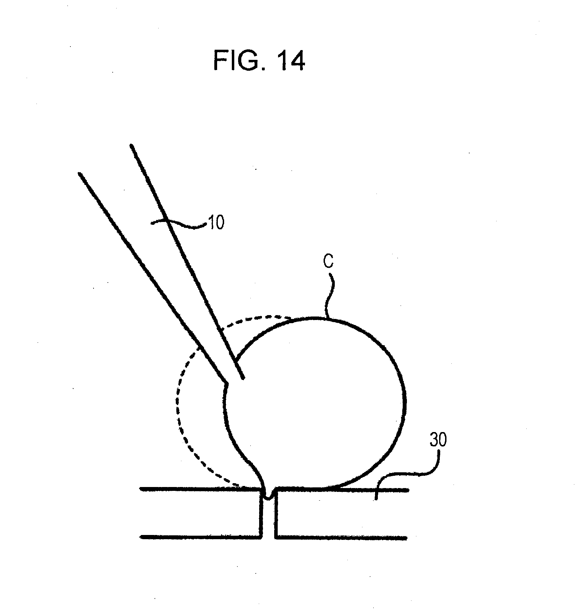

When the position of the cell C is fixed by suction, it is necessary to properly adjust the relationship between the size of the cell and the size of the through-hole. More specifically, if the size of the through-hole in the cell capturing plate 30 is too large with respect to the size of the cell C as shown, by way of example, in Fig. 13, there is a fear that the cell C will be forced through the through-hole by the suction. Also, if the size of the through-hole in the cell capturing plate 30 is too small with respect to the size of the cell C as shown, by way of example, in Fig. 14, there is a fear that the position of the cell C cannot be positively fixed and the cell C can be moved when the capillary needle 10 contacts the cell C.

In view of the above-mentioned problem, Japanese laid-open patent publication 2005-318851, for example, describes a cell capturing plate in which a through-hole having a small diameter is bored in the plate and a recess having a large diameter is formed to surround the through-hole. Stated another way, in the cell capturing plate described therein, the through-hole is formed at a center of the recess which has a circular flat surface positioned at a lower level than its surroundings. According to the cell capturing plate thus constructed, at the same time as when the cell is attracted to the cell capturing plate by suction via the through-hole, the cell position is positively fixed by the presence of the recess.

However, cells as microinjection targets have various sizes and all the target cells cannot be always captured depending on cell diameters even with the arrangement including the recess which surrounds the through-hole bored therein. More specifically, because the diameter of the recess in the above-described Reference corresponds to about 80% of the cell diameter, the diameter of the cell capable of being captured by the recess is necessarily limited by the recess diameter. Accordingly, different cell capturing plates need to be selectively employed depending on the diameters of the microinjection target cells, and efficiency of the microinjection deteriorates.

Further, even for the same type of cells, diameters of individual cells often differ to a large extent. In such a case, a sufficient capturing force is not exerted on some of the captured cells which do not fit the recess diameter. Hence, the injection is more apt to fail and efficiency of the injection deteriorates.

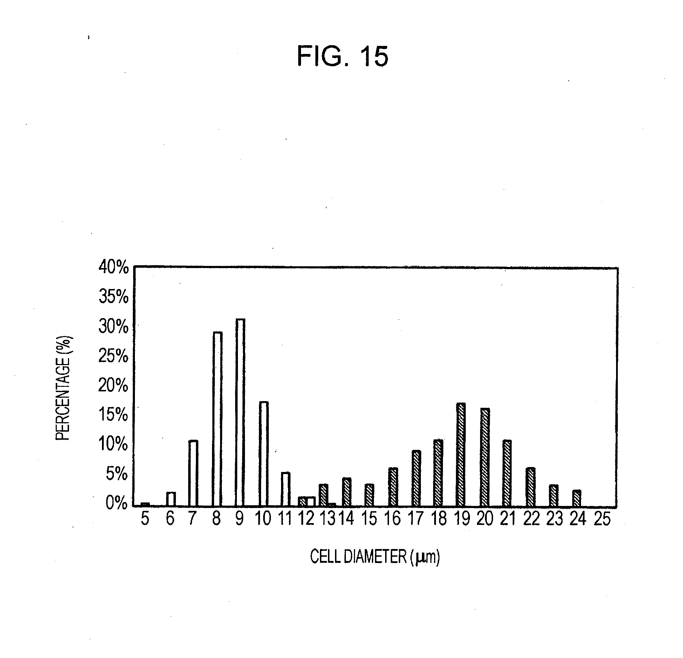

To explain the above-mentioned problems in more detail, Fig. 15 shows histograms indicating diameters of two types of cells. In Fig. 15, a histogram representing the first cell type is indicated by hollow bars, and a histogram representing the second cell type is indicated by hatched bars. As shown in Fig. 15, diameters of the first type of cells are distributed primarily over the range of 7 - 10 µm, and diameters of the second type of cells are distributed primarily over the range of 13 - 23 µm. There is a difference of about 11 µm between respective average values of the two types of cells. Accordingly, it may be difficult to capture those two species of cells by using the same cell capturing plate.

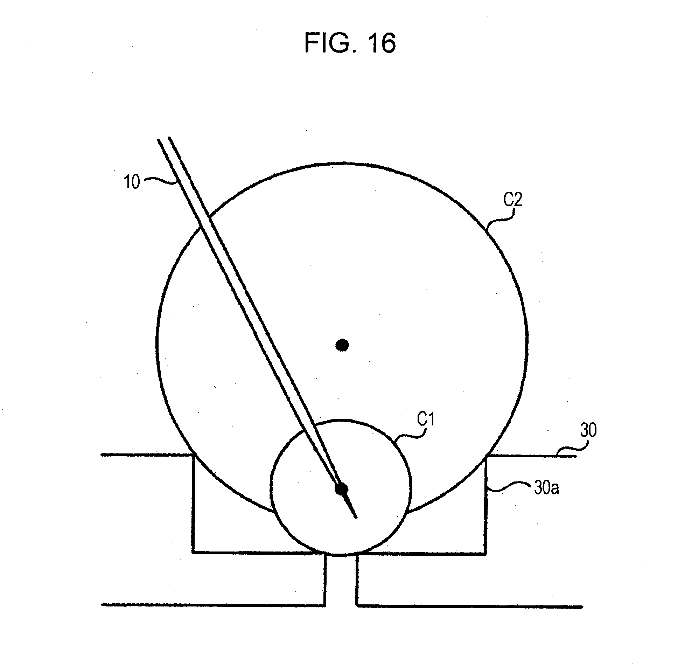

More specifically, for example, when a cell capturing plate suitable for the second type of cells having larger diameters is used, the second type of cell C2 is captured, as shown in Fig. 16, by a recess 30a which is formed in a cell capturing plate 30 and which surrounds a through-hole bored in the plate 30. On the other hand, the first type of cell C1 entirely comes into the recess 30a of the cell capturing plate 30. Therefore, the recess 30a does not fulfill the function of capturing the cell C1, and just a small capturing force corresponding to the cross-sectional area of the through-hole is exerted on the cell C1. Further, as seen from the histogram shown in Fig. 15, the diameters of the second type of cells are distributed over a wide range in spite of the same cell type. Accordingly, even when the microinjection is performed on only the second type of cells, a sufficient capturing force is not exerted on many of the cells captured by the cell capturing plate.

In addition, as shown in Fig. 16, the longitudinal direction of the capillary needle 10 is inclined at a certain angle with respect to the vertical direction. The reason is that an illuminator for illuminating the cell and other devices are disposed in an area vertically above the cell as the microinjection target, the capillary needle 10 and a mechanism for moving the capillary needle 10 cannot be disposed in the area vertically above the target cell. In such an arrangement, when the movement of the capillary needle 10 is controlled such that the distal end of the capillary needle 10 passes the center of the cell C1 having the smaller diameter in the state of the capillary needle 10 being projected, the path along which the distal end of the capillary needle 10 moves is largely deviated from the center of the cell C2 having the larger diameter, as is apparent from Fig. 16.

Accordingly, when a medical fluid is injected into the cell C2 having the larger diameter, the distal end of the capillary needle 10 reaches a position deviated from the center of the cell C2, and reliable performance of the microinjection is not ensured. In other words, the medical fluid is injected to a position located in an outer edge portion of the cell, and the effect of the injected medical fluid is not developed in some cases. Further, there is a fear that, when the capillary needle 10 is projected, the distal end of the capillary needle 10 contacts the end of the cell C2 and pushes the cell C2 away from the recess 30a.

It is desirable to provide a cell capturing plate, a microinjection apparatus, and a method of producing the cell capturing plate, which can capture cells having different diameters and can reliably inject a medical fluid into each of the captured cells.

In accordance with an aspect of the present invention, there is provided a cell capturing plate, comprising: a flat surface; a capturing hole having an opening formed in a first side of the flat surface in a direction substantially perpendicular to the flat surface for supporting a cell, and a through-hole extending from the opening to a second, opposite side of the flat surface, wherein an area of a first cross section parallel to the flat surface near the opening is larger than an area of a second cross-section parallel to the flat surface near the through-hole and a linear line connecting centers of the first and second sides is oblique relative to the flat surface.

**[0016A]** According to another aspect of the present invention, there is provided a microinjection apparatus having a capillary needle being projectable along a longitudinal axis of the needle and delivering a material at predetermined timing for injection into a cell, and a cell capturing plate having a flat surface and capturing injection target cells, the cell capturing plate comprising: a capturing hole having an opening formed in a first side of the flat surface in a direction substantially perpendicular to the flat surface for supporting the cell, and a through-hole extending from the opening to a second, opposite side of the flat surface, wherein an area of a first cross-section parallel to the flat surface near the opening is larger than an area of a second cross-section parallel to the flat surface near the through-hole and a linear line connecting centers of the first and second cross-sections is substantially parallel to the longitudinal axis of the capillary needle.

**[0016B]** According to another aspect of the present invention, there is provided a method of producing a cell capturing plate having a flat surface and capturing a cell towards which a capillary needle is projectable along a longitudinal axis of the needle, the method comprising the steps of: forming, by etching, a through-hole extending from a first side of the flat surface to a second, opposite side of the flat surface; forming, by etching, a first cylindrical portion which surrounds the through-hole and which is located nearer to the second side of the flat surface; and forming, by etching, a second cylindrical portion, which surrounds the first cylindrical portion, wherein a linear line connecting a center of an opening formed in the first side of the flat surface and a center of an opening of the first cylindrical portion, which is formed at a bottom area of the second cylindrical portion, is substantially parallel to the longitudinal axis of the capillary needle.

The accompanying drawings, which are incorporated in and constitute a part of the specification, illustrate embodiments of the invention and, together with the description, serve to explain the principles of the invention.

In the Drawings:
Fig. 1 is a block diagram showing the construction of an automatic microinjection apparatus according to a first embodiment.
Fig. 2 is a plan view of a cell capturing plate according to the first embodiment.
Fig. 3 is a sectional view of the cell capturing plate according to the first embodiment.
Fig. 4 is a plan view of a capturing hole according to the first embodiment.
Fig. 5 is a sectional view of the capturing hole according to the first embodiment.
Fig. 6 is a sectional view of the capturing hole according to the first embodiment.
Fig. 7 is a sectional view showing an example of the construction of a multi-stepped capturing hole.
Fig. 8 is a flowchart showing the injection operation according to the first embodiment.
Fig. 9 illustrates a method of producing the cell capturing plate according to the first embodiment.
Fig. 10 is a plan view of a capturing hole according to a second embodiment.
Fig. 11 is a sectional view of the capturing hole according to the second embodiment.
Fig. 12 illustrates one example of a cell capturing method when the injection is performed.
Fig. 13 illustrates one example of the state of a captured cell.
Fig. 14 illustrates another example of the state of a captured cell.
Fig. 15 indicates an example of distribution of cell diameters.
Fig. 16 illustrates the captured state of cells having different diameters.

Reference will now be made in detail to the embodiments, examples of which are illustrated in the accompanying drawings, wherein like reference numerals refer to the like elements throughout. The embodiments are described below to explain the present invention by referring to the figures.

According to the present invention a cross-section of a capturing hole formed in a capturing plate, as viewed perpendicularly to a direction in which the capturing hole is opened, has a size gradually reduced and a center position gradually increasingly eccentric from a cross-section center at an opening of the capturing hole as the cross-section approaches a through-hole, and that a linear line connecting the cross-section center at the opening of the capturing hole for capturing a cell and a cross-section center near the through-hole is oblique relative to the capturing plate or substantially parallel to a longitudinal axis of a capillary needle. Embodiments of the present invention will be described in detail below with reference to the drawings.

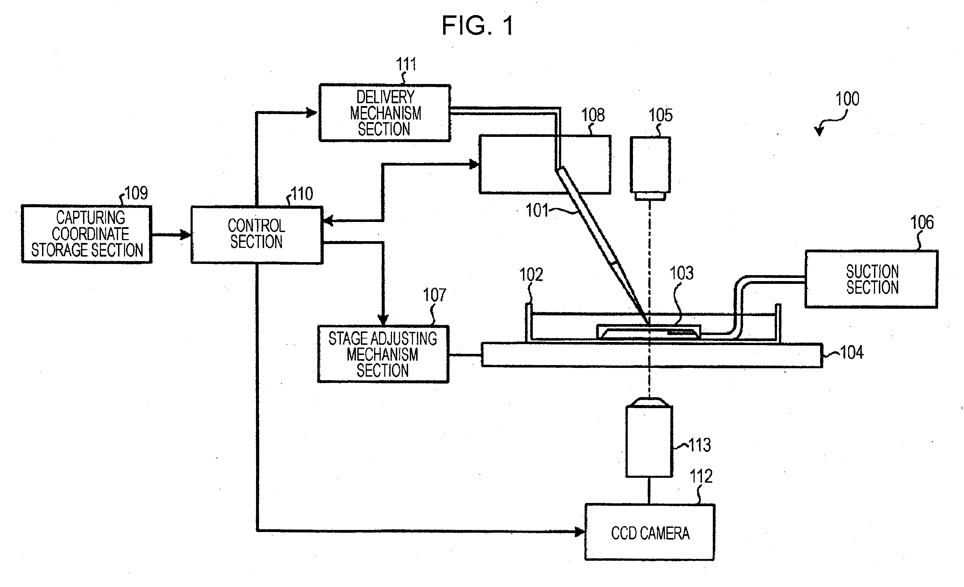

Fig. 1 is a block diagram showing the construction of an automatic microinjection apparatus 100 according to a first embodiment of the present invention. The automatic microinjection apparatus 100, shown in Fig. 1, includes a capillary needle 101, a petri dish 102, a cell capturing plate 103, a stage 104, an illuminator 105, a suction section 106, a stage adjusting mechanism section 107, a capillary-needle moving mechanism section 108, a capturing coordinate storage section 109, a control section 110, a delivery mechanism section 111, a CCD (Charge Coupled Device) camera 112, and an inverted microscope 113.

The capillary needle 101 is mounted to the capillary-needle moving mechanism section 108 such that the capillary needle 101 is movable over a horizontal plane and it can be projected and withdrawn in its longitudinal direction. The capillary needle 101 delivers a medical fluid held therein when a delivery pressure is applied from the delivery mechanism section 111.

The petri dish 102 is a substantially cylindrical container with its bottom surface having a diameter larger than a height, and is filled with a buffer in which cells can live. The petri dish 102 is placed on the stage 104 and is moved on a horizontal plane together with the stage 104.

The cell capturing plate 103 is put at a center of the petri dish 102 and captures the cells in the buffer at positions of capturing holes which are formed in a flat plate surface thereof parallel to the bottom surface of the petri dish 102. A space defined by a leg of the cell capturing plate 103 and the bottom surface of the petri dish 102 is enclosed, and two spaces partitioned by the cell capturing plate 103 within the petri dish 102 are communicated with each other through only the capturing holes formed in the cell capturing plate 103. In other words, only through-holes bored within the capturing holes in the cell capturing plate 103 serve as passages for the buffer to be sucked. Further details regarding the cell capturing plate 103 will be described later.

The stage 104 is fixed to the stage adjusting mechanism section 107 and can receive the petri dish 102 placed on an upper surface thereof. The stage 104 is moved under control of the stage adjusting mechanism section 107 such that the microinjection target cell captured on the cell capturing plate 103 is positioned vertically under the illuminator 105 and vertically above a lens of the inverted microscope 113. In addition, the stage 104 is made of a transparent material or has a photographing hole in its central portion where the cell capturing plate 103 is located, so that the CCD camera 112 can take an image of the cell on the cell capturing plate 103.

The illuminator 105 is a light source for illuminating the cells captured by the cell capturing plate 103 and is disposed vertically above the lens of the inverted microscope 113. The position of the illuminator 105 is always held fixed.

The suction section 106 sucks the buffer from the lower side of the cell capturing plate 103 nearer to the bottom surface of the petri dish 102 to generate a negative pressure state in the lower side of the cell capturing plate 103 nearer to the bottom surface of the petri dish 102 relative to the upper side of the cell capturing plate 103 where the cells are present.

The stage adjusting mechanism section 107 moves the stage 104 on the horizontal plane in accordance with an instruction from the control section 110. More specifically, the stage adjusting mechanism section 107 moves the stage 104 such that the coordinate position of the capturing hole in the cell capturing plate 103 where the cell is captured is located vertically under the illuminator 105 and vertically above the lens of the inverted microscope 113.

The capillary-needle moving mechanism section 108 moves the capillary needle 101 on the horizontal plane such that, when the injection is performed, the distal end of the capillary needle 101 is positioned vertically under the illuminator 105 and vertically above the lens of the inverted microscope 113. Further, after completion of the movement of the capillary needle 101 on the horizontal plane, the capillary-needle moving mechanism section 108 projects the distal end of the capillary needle 101 forwards in its longitudinal direction and then withdraws the distal end of the capillary needle 101 backwards in its longitudinal direction. At that time, the capillary-needle moving mechanism section 108 notifies the displacement of the capillary needle 101 in the longitudinal direction to the control section 110.

The capturing coordinate storage section 109 stores respective coordinates of the capturing holes formed in the cell capturing plate 103 which is placed in the petri dish 102. In other words, the capturing coordinate storage section 109 previously stores the respective capturing coordinates at which the cells are captured.

The control section 110 reads the coordinates of one capturing hole from the capturing coordinate storage section 109 and instructs the read coordinates to the stage adjusting mechanism section 107 so that the capturing hole corresponding to the read coordinates is positioned vertically under the illuminator 105 and vertically above the lens of the inverted microscope 113. Also, after the position of the stage 104 has been adjusted, the control section 110 instructs the capillary-needle moving mechanism section 108 to perform fine adjustment of the position of the stage 104.

Further, the control section 110 receives, from the capillary-needle moving mechanism section 108, the notification of the displacement of the capillary needle 101 in the longitudinal direction and instructs the delivery mechanism section 111 to deliver the medical fluid later than when the capillary needle 101 is maximally projected. More specifically, the control section 110 instructs the delivery mechanism section 111 to deliver the medical fluid when the distal end of the capillary needle 101 is lifted to a predetermined height after the capillary needle 101 has been maximally projected and its distal end has come closest to the cell capturing plate 103.

In response to the instruction from the control section 110 to deliver the medical fluid, the delivery mechanism section 111 applies the delivery pressure to the interior of the capillary needle 101, thereby delivering the medical fluid from the distal end of the capillary needle 101.

The CCD camera 112 takes a cell image magnified by the inverted microscope 113, thus displaying and photographing the state that the medical fluid is injected into the cell captured on the cell capturing plate 103. Also, the CCD camera 112 takes an image indicating a change that has occurred in the cell after injecting the medical fluid.

The inverted microscope 113 is installed at a position capable of observing an area vertically under the illuminator 105 in an enlarged scale. Namely, the inverted microscope 113 magnifies an image of the vicinity of the distal end of the capillary needle 101 and the cell captured by the cell capturing plate 103.

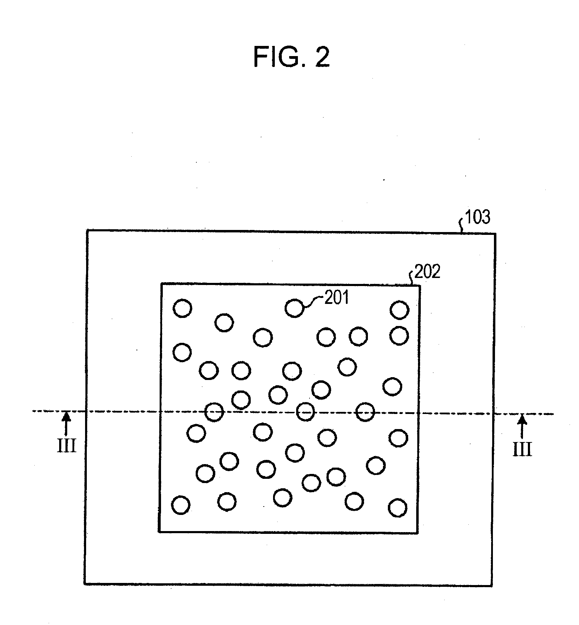

Fig. 2 is a plan view showing the construction of the cell capturing plate 103 according to the first embodiment. As shown in Fig. 2, a plurality of capturing holes 201 are formed in a central flat surface portion 202 of the cell capturing plate 103. The capturing holes 201 may be regularly arrayed in the flat surface portion 202. By arranging the capturing holes 201 at random as shown in Fig. 2, however, the flat surface portion 202 can be made less apt to cause cracks, thus ensuring that the flat surface portion 202 has sufficient strength. In this embodiment, particularly, because one side of the flat surface portion 202 (i.e., the side closer to the bottom surface of the petri dish 102) is placed under a negative pressure state and pressure is exerted on the flat surface portion 202, the flat surface portion 202 is required to have a certain degree of strength.

The coordinates of each capturing hole 201 in the flat surface portion 202 are previously stored in the capturing coordinate storage section 109. Accordingly, by moving the stage 104 on the horizontal plane such that the coordinate position of the capturing hole 201 is located vertically under the illuminator 105 and vertically above the lens of the inverted microscope 113, the cell captured in the capturing hole 201 can be set as the microinjection target cell. The opening diameter of the capturing hole 201 is desirably about 70 - 80% of the diameter of the largest cell as the microinjection target. In one practical example, when the largest cell diameter is about 16 µm, the opening diameter of the capturing hole 201 is desirably about 12 µm.

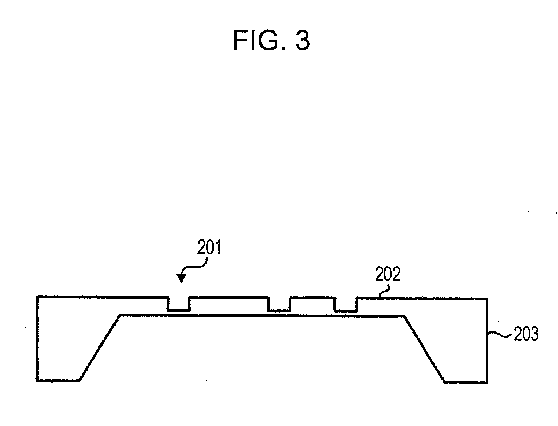

Fig. 3 is a sectional view showing a cross-section taken along a line III-III in Fig. 2. As shown in Fig. 3, a leg 203 for supporting the flat surface portion 202 of the cell capturing plate 103 is formed in a surrounding relation to the flat surface portion 202. The leg 203 is held in close contact with the bottom surface of the petri dish 102 such that only the through-holes bored within the capturing holes 201 serve as passages for the surrounding buffer. More specifically, though not shown in Fig. 3, the through-holes are bored in bottom surfaces of the capturing holes 201. When the lower side of the flat surface portion 202 located on the same side as the leg 203 is subjected to negative pressure, the buffer present in the upper side of the flat surface portion 202 opposite to the leg 203 is sucked toward the lower side of the flat surface portion 202 located on the same side as the leg 203 via the through-holes bored in the bottom surfaces of the capturing holes 201. At that time, with the suction of the buffer, the cells in the buffer are also attracted into the capturing holes 201 and are captured in the capturing holes 201.

The depth from an upper surface of the flat surface portion 202 to the bottom surface of the capturing hole 201 is set to such a size as enabling the cell to be positively fixed, and it is, e.g., about 7 - 8 µm. Further, one or more through-holes are bored in the bottom surface of the capturing hole 201 to penetrate the flat surface portion 202. Therefore, the thickness of the flat surface portion 202 is required to be larger than the depth of the capturing hole 201, and it is set to, e.g., about 10 µm.

Though not shown in Fig. 3 for the sake of simplifying the drawing, the capturing hole 201 is formed such that a cross-sectional area in a direction perpendicular to the direction in which the capturing hole is opened is gradually reduced, and that a linear line connecting the cross-section center at the opening of the capturing hole and a cross-section center near the bottom surface of the capturing hole is substantially parallel to a longitudinal axis of the capillary needle 101. Such a shape of the capturing hole 201 is described in more detail below.

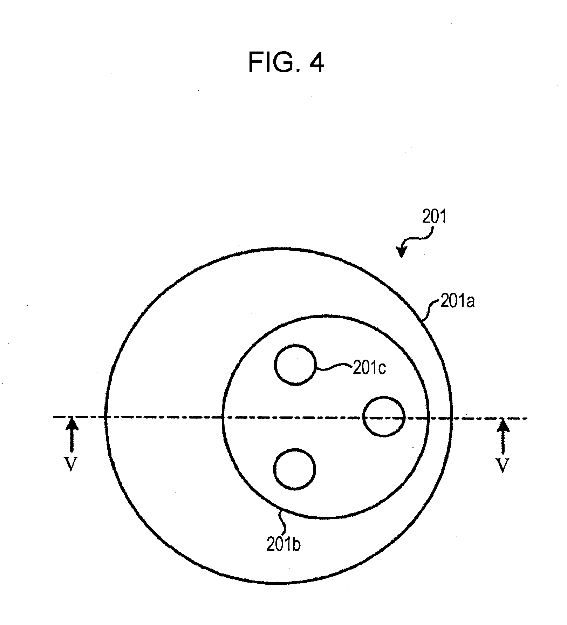

Fig. 4 is a plan view showing the construction of the capturing hole 201 according to the first embodiment. As shown in Fig. 4, an upper-step cylindrical recess 201 a for capturing the largest cell and a lower-step cylindrical recess 201 b for capturing a cell smaller than the largest cell are formed within the capturing hole 201. More specifically, at a bottom surface of the upper-step cylindrical recess 201 a, the lower-step cylindrical recess 201 b is formed such that a bottom surface of the lower-step cylindrical recess 201 a has a smaller area than the bottom surface of the upper-step cylindrical recess 201 a and a center of the bottom surface of the upper-step cylindrical recess 201a is not coincident with a center of the bottom surface of the lower-step cylindrical recess 201 b. Thus, as shown in Fig. 4, two circles corresponding to the bottom surface of the upper-step cylindrical recess 201 a and the bottom surface of the lower-step cylindrical recess 201 b are eccentric from each other as viewed in a plan.

While this embodiment is described as forming two-stage recesses, i.e., the upper-step cylindrical recess 201 a and the lower-step cylindrical recess 201 b, in the capturing hole 201, three or more cylindrical recesses may be formed within the capturing hole 201. Also when three or more cylindrical recesses are formed, a bottom surface of the cylindrical recess in the lower-step has a smaller area and has a center located more eccentric. Stated another way, the center of the bottom surface of the cylindrical recess in the lower-step is positioned farther away from the capillary needle 101.

The diameter of the bottom surface of the upper-step cylindrical recess 201 a is, as described above, desirably about 70 - 80% of the diameter of the largest cell as the microinjection target. Similarly, the diameter of the bottom surface of the lower-step cylindrical recess 201 b is desirably about 70 - 80% of the diameter of the smallest cell as the microinjection target. Thus, when multistep cylindrical recesses are formed within the capturing hole 201, the diameter of the bottom surface of each cylindrical recess is desirably about 70 - 80% of the diameter of the cell as the capturing target. In other words, the cell having a size satisfying the above-described relationship with respect to the diameter of the bottom surface of the cylindrical recess is captured by the corresponding cylindrical recess.

Further, a plurality of through-holes 201 c is formed in the bottom surface of the lower-step cylindrical recess 201 b. The through-holes 201 c penetrate the flat surface portion 202 and serve as passages for the buffer when the lower side of the flat surface portion 202 nearer to the bottom surface of the petri dish 102 is subjected to negative pressure. Note that the number of the through-holes 201c formed within the capturing hole 201 may be one. Also, when the plurality of through-holes 201 c is formed within the capturing hole 201, a layout of the through-holes 201 c is not limited to that shown in Fig. 4. In this embodiment, an attractive force exerted on the cell is increased by forming the plurality of through-holes 201 c in one capturing hole 201. The diameter of the through-hole 201 c is sufficiently small with respect to the diameter of the cell to be captured, and it is, e.g., about 1 - 2 µm. As an alternative, the diameter of the through-hole 201 c may be set to about 10 - 20% of the smallest cell diameter.

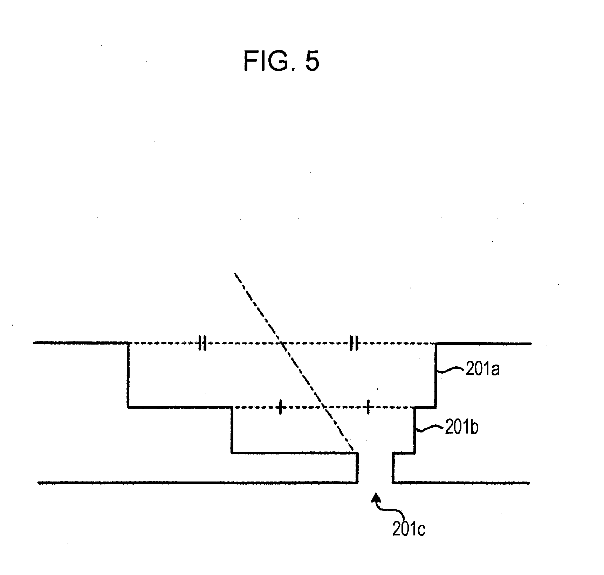

Fig. 5 is a sectional view showing a cross-section taken along a line V-V in Fig. 4. As shown in Fig. 5, a linear line (indicated by a one-dot-chain line in Fig. 5) connecting a center of the cross-section at the opening of the upper-step cylindrical recess 201 a and a center of the cross-section at the opening of the lower-step cylindrical recess 201 b is inclined relative to the vertical direction. Further, that linear line is substantially parallel to the longitudinal axis of the capillary needle 101. In addition, the bottom surface of the lower-step cylindrical recess 201 b has a smaller area than that of the upper-step cylindrical recess 201a, and the capturing hole 201 has such an entire shape that steps are formed in an inner wall of an eccentric funnel tapering toward the through-hole 201 c. In other words, the opening of the lower-step cylindrical recess 201 b is entirely included in the bottom surface of the upper-step cylindrical recess 201 a and, when looking at the capturing hole 201 from the opening side, the entire bottom surface of the lower-step cylindrical recess 201 b can be seen as shown in Fig. 4.

According to this embodiment, as described above, since the multistep cylindrical recesses are formed within the capturing hole 201, cells of corresponding sizes can be positively captured by the respective cylindrical recesses, and cells having different diameters can be captured by one cell capturing plate 103. Also, with such an arrangement that the linear line connecting the centers of the openings of the multistep cylindrical recesses is substantially parallel to the longitudinal axis of the capillary needle 101, when the capillary needle 101 is projected, the distal end of the capillary needle 101 reaches near the center of each of the cells captured by the cylindrical recesses. As a result, the medical fluid can be reliably injected into each of the captured cells.

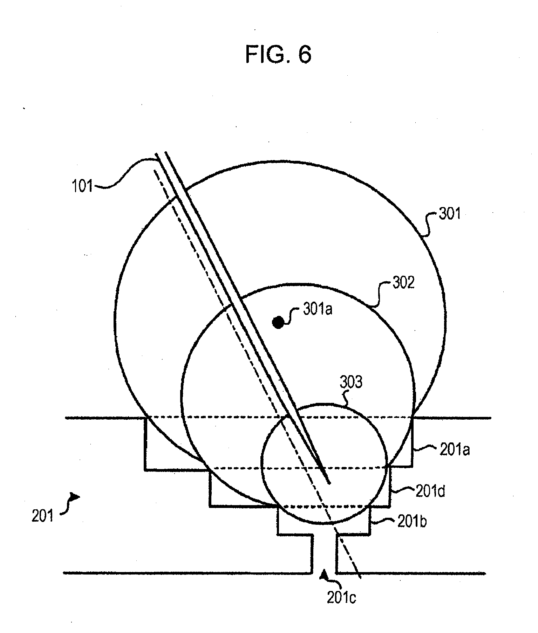

An exemplary state in injecting the medical fluid into the cell captured by the capturing hole 201 according to this embodiment will be described below with reference to Fig. 6. Fig. 6 is a sectional view showing an example of the construction of the capturing hole 201 according to this embodiment. The capturing hole 201, shown in Fig. 6, is formed so as to have cylindrical recesses in three stages. Thus, the capturing hole 201 includes a middle-step cylindrical recess 201 d in addition to the upper-step cylindrical recess 201 a and the lower-step cylindrical recess 201 b.

The upper-step cylindrical recess 201 a is formed to capture a large-sized cell 301. Similarly, the middle-step cylindrical recess 201 d and the lower-step cylindrical recess 201 b are formed to capture a medium-sized cell 302 and a small-sized cell 303, respectively. Bottom surface areas of those three cylindrical recesses are gradually reduced toward the lower-step, and a linear line (indicated by a one-dot-chain line in Fig. 6) connecting respective centers of openings of the cylindrical recesses is substantially parallel to the longitudinal axis of the capillary needle 101.

The large-sized cell 301, the medium-sized cell 302, and the small-sized cell 303 corresponding to the respective cylindrical recesses are supported at steps formed by peripheral edges of the openings of the corresponding cylindrical recesses and are positively fixed in the capturing hole 201 by a suction force that is generated with negative pressure produced below the through-hole 201c.

Further, the linear line connecting the centers of the openings of the three cylindrical recesses is substantially parallel to the longitudinal axis of the capillary needle 101, thus providing a multi-stepped shape wherein the centers of the bottom surfaces of the cylindrical recesses are located gradually increasingly eccentric. Therefore, when the capillary needle 101 is projected, the distal end of the capillary needle 101 reaches near the center of each of the cells captured by the cylindrical recesses. Stated another way, by adjusting the stage adjusting mechanism section 107 and the capillary-needle moving mechanism section 108 such that the distal end of the capillary needle 101 reaches near the center of the small-sized cell 303 captured by the lower-step cylindrical recess 201 b, the distal end of the capillary needle 101 passes near a center 301a of the large-sized cell 301 captured by the upper-step cylindrical recess 201 a.

Accordingly, the medical fluid delivered from the distal end of the capillary needle 101 can be reliably injected into the cell. Also, when the capillary needle 101 is projected, the distal end of the capillary needle 101 avoids contacting the end of the cell and push the captured cell away from the capturing hole. It is hence ensured that the distal end of the capillary needle 101 penetrates the cell membrane of the captured cell and the medical fluid is injected into the captured cell.

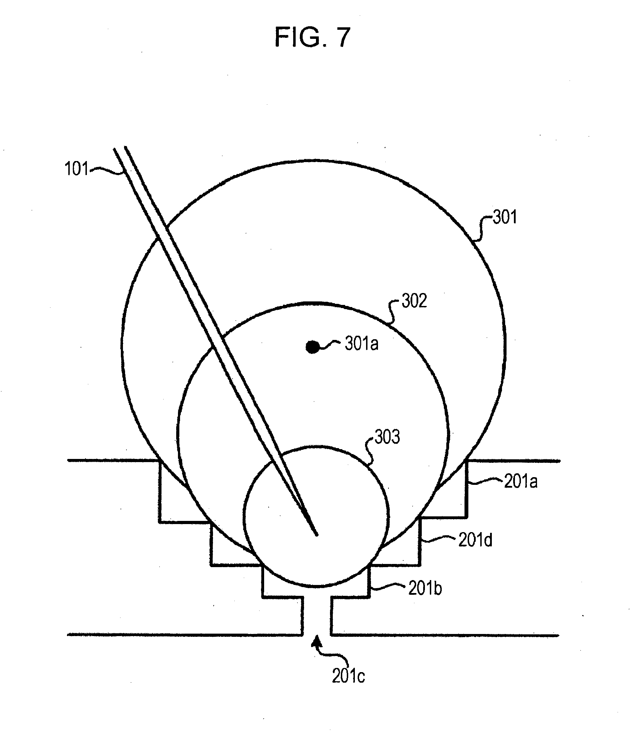

In contrast, if the cylindrical recesses were formed so as to have concentric bottom surfaces as shown in Fig. 7, for example, the large-sized cell 301, the medium-sized cell 302, and the small-sized cell 303 can be captured by the respective cylindrical recesses. In such a case, however, when the capillary needle 101 is projected, the distal end of the capillary needle 101 cannot reach near the center of each of all the cells captured by the cylindrical recesses. More specifically, when the stage adjusting mechanism section 107 and the capillary-needle moving mechanism section 108 are adjusted such that the distal end of the capillary needle 101 reaches near the center of the small-sized cell 303 captured by the lower-step cylindrical recess 201 b, the distal end of the capillary needle 101 passes a position largely away from the center 301 a of the large-sized cell 301 captured by the upper-step cylindrical recess 201a.

In comparison with the case of the cylindrical recesses having the eccentric bottom surfaces according to the present invention, therefore, reliability in injection of the medical fluid into the cell is reduced with the conventional apparatus and the captured cell is pushed away from the capturing hole at a higher possibility with the movement of the distal end of the capillary needle 101.

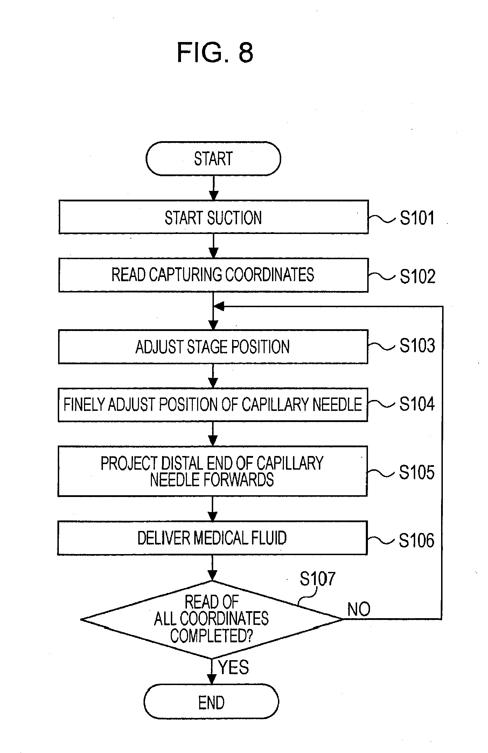

Next, the injection operation using the cell capturing plate 103 having the above-described construction will be described with reference to a flowchart shown in Fig. 8.

At start of the injection, the cell capturing plate 103 is placed in the petri dish 102 and a buffer in which many cells are living is added to the petri dish 102. In such a state, the suction section 106 starts the suction from the lower side of the cell capturing plate 103 nearer to the bottom surface of the petri dish 102 (step S101). With the start of the suction, negative pressure is generated in the lower side of the cell capturing plate 103 nearer to the bottom surface of the petri dish 102, thus causing the buffer in the petri dish 102 to pass through the through-holes 201 c in the cell capturing plate 103 and to flow into the lower side of the cell capturing plate 103 nearer to the bottom surface of the petri dish 102. Correspondingly, the cells in the buffer are also sucked toward the through-holes 201c and are captured by the capturing holes 201. Thus, the cells are fixed at respective coordinate positions of the capturing holes 201 in the cell capturing plate 103.

At that time, since the multistep cylindrical recesses are formed in each of the capturing holes 201, the cells having different sizes are fixed in the capturing holes 201 at the cylindrical recesses corresponding to the cell sizes. Accordingly, even when the microinjection is performed on the type of cell that has a large variation in cell size, the sizes of the captured cells are well balanced. Also, even when the microinjection is performed on different types of cells, the cell capturing plate 103 is not required to be replaced whenever the target cell type is changed.

On the other hand, the control section 110 reads the respective coordinates of all the capturing holes 201 from the capturing coordinate storage section 109 (step S102). Because the cells are captured in the capturing holes 201 as described above, the coordinates of each capturing hole 201 means the capturing coordinates at which the cell is captured. After the control section 110 reads the respective capturing coordinates of all the capturing holes 201, one set of capturing coordinates is notified from the control section 110 to the stage adjusting mechanism section 107, whereby the stage adjusting mechanism section 107 is instructed to move the position represented by the capturing coordinates to a target position of the microinjection. In other words, the stage adjusting mechanism section 107 is requested to adjust the position of the stage 104 so that the position represented by the capturing coordinates where the cell is captured is located vertically under the illuminator 105 and vertically above the lens of the inverted microscope 113.

Responsively, the position of the stage 104 is adjusted by the stage adjusting mechanism section 107 (step S103). More specifically, the stage 104 is moved on the horizontal plane by the stage adjusting mechanism section 107, and the petri dish 102 and the cell capturing plate 103 on the stage 104 are fixed to the position as instructed by the control section 110. Further, the horizontal position of the capillary needle 101 is finely adjusted by the capillary-needle moving mechanism section 108 so that the distal end of the capillary needle 101 is positioned vertically under the illuminator 105 when the medical fluid is delivered (step 104).

After the respective horizontal positions of the stage 104 and the capillary needle 101 have been adjusted, the distal end of the capillary needle 101 is projected forwards in the longitudinal direction thereof by the capillary-needle moving mechanism section 108 (step S105). The cell is captured by the capturing hole 201 in the cell capturing plate 103 on an extension of the capillary needle 101 in the longitudinal direction thereof. Accordingly, when the capillary needle 101 is projected, the distal end of the capillary needle 101 contacts the cell. At that time, since the multistep cylindrical recesses having the eccentric bottom surfaces are formed within the capturing hole 201 in the cell capturing plate 103, the distal end of the capillary needle 101 contacts near the center of the cell membrane and penetrates the cell membrane without pushing the cell away from the capturing hole.

When the distal end of the capillary needle 101 penetrates the cell membrane and reaches a predetermined position, the control section 110 instructs the delivery mechanism section 111 to deliver the medical fluid. In response to the instruction, the delivery mechanism section 111 applies a delivery pressure to the interior of the capillary needle 101, whereby the medical fluid is delivered from the distal end of the capillary needle 101 (step S106). The timing of delivering the medical fluid may be set to the timing at which the distal end of the capillary needle 101 is projected to the predetermined position. Alternatively, the delivering timing may be set to the timing at which the distal end of the capillary needle 101 is lifted up to a predetermined position after being maximally projected. In the latter case, since the distal end of the capillary needle 101 is first maximally projected, the distal end of the capillary needle 101 can certainly penetrate the pliable cell membrane. Further, since the medical fluid is delivered during a lift-up stroke of the capillary needle 101, a space for receiving the delivered medical fluid is formed within the cell.

When the capillary needle 101 is lifted up to its initial position after the medical fluid has been delivered from the distal end of the capillary needle 101, the control section 110 determines whether the injection has been completed for all the capturing coordinates read from the capturing coordinate storage section 109 (step S107). If there remains one or more capturing coordinates for which the injection is not yet completed (No in step S107), the above-described processing is repeated from the movement of the stage 104 by the stage adjusting mechanism section 107. In such a manner, the injection is completed for the cells captured at the positions represented by all the capturing coordinates.

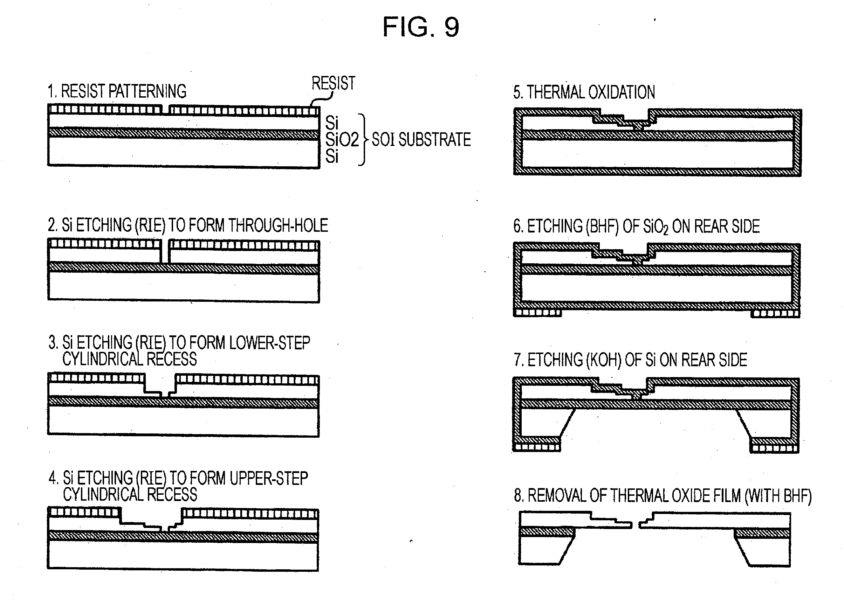

Next, a method of producing the cell capturing plate 103 according to this embodiment will be described with reference to Fig. 9. Fig. 9 illustrates producing steps 1-8 of the cell capturing plate 103.

The cell capturing plate 103 is produced by using an SOI (Silicon On Insulator) substrate in which a silicon oxide film (indicated by oblique hatching in Fig. 9) is sandwiched between two active silicon layers. First, the shape of the capturing hole 201 is transferred to the SOI substrate by resist patterning (resist is indicated by vertical hatching in Fig. 9) (production step 1). Then, the through-hole 201 c is formed in the active silicon layer with a thickness of about 10 µm by RIE (Reactive Ion Etching) (production step 2). Similarly, the lower-step cylindrical recess 201 b and the upper-step cylindrical recess 201a are formed by RIE (production steps 3 and 4). Through the above steps, the shape of the capturing hole 201 in the cell capturing plate 103 is prepared.

The SOI substrate formed with the capturing hole 201 is entirely subjected to thermal oxidation to be protected by a silicon film (production step 5). Thereafter, a portion of the SOI substrate which corresponds to the leg 203 is masked by a resist and the silicon oxide film positioned under the flat surface portion 202 is etched away with buffered hydrofluoric acid (BHF) (production step 6). Subsequently, the active silicon layer positioned under the flat surface portion 202 is anisotropically etched away with a solution of potassium hydroxide (KOH) (production step 7). Finally, the entire silicon oxide film is removed with buffered hydrofluoric acid (BHF), whereby the cell capturing plate 103 is completed (production step 8).

Thus, in the cell capturing plate 103 according to this embodiment, since the capturing hole 201 is formed by the etching called RIE, the very small through-hole 201 c and each cylindrical recess can be formed with high accuracy. As a result, the multistep cylindrical recesses having respective centers of their bottom surfaces arranged to lie substantially parallel to the longitudinal axis of the capillary needle 101 are formed in accurate positions. Hence, the cell capturing plate 103 can capture the cells having different diameters and can ensure reliable injection of the medical fluid into each of the captured cells.

According to this embodiment, as described above, the multistep cylindrical recesses are formed within the capturing hole for capturing the cell with suction via the through-hole bored in the cell capturing plate, the multistep cylindrical recesses having the bottom surface areas gradually reduced toward the through-hole, and the linear line connecting the centers of respective cross-sections of the multistep cylindrical recesses, as viewed perpendicularly to the direction in which the multistep cylindrical recesses are each opened, is substantially parallel to the longitudinal axis of the capillary needle. Therefore, the cells having different sizes can be captured by the corresponding cylindrical recesses, and when the capillary needle is projected, the distal end of the capillary needle can be moved to reach near the center of each captured cell. Consequently, it is possible to capture the cells having different diameters and to reliably inject the medical fluid into each of the captured cells.

In a second embodiment of the present invention no steps are formed within the capturing hole and the capturing hole is formed in the shape of a funnel eccentrically tapering toward the through-hole.

Since the construction of the automatic microinjection apparatus and the basic structure of the cell capturing plate 103 according to the second embodiment are similar to those in the first embodiment, a description thereof is omitted here. In the second embodiment, only the shape of the capturing hole 201 formed in the cell capturing plate 103 differs from that in the first embodiment.

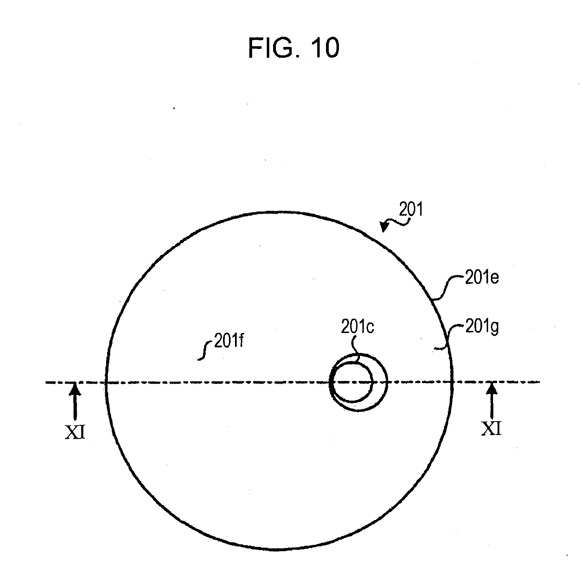

Fig. 10 is a plan view showing the construction of the capturing hole 201 according to the second embodiment. As shown in Fig. 10, the capturing hole 201 is formed such that its inner surfaces 201f and 201g extending from an opening 201e to a through-hole 201 c have no steps and two circles corresponding to the opening 201 e of the capturing hole 201 and the bottom surface thereof in which the through-hole 201 c is formed are eccentric from each other. In other words, the inner surface 201f of the capturing hole 201 on the side nearer to the capillary needle 101 is larger than the inner surface 201 g of the capturing hole 201 on the side opposed to the capillary needle 101.

The diameter of the opening 201 e of the capturing hole 201 is desirably 70 - 80% of the largest cell as the microinjection target. Also, the diameter of the bottom surface of the capturing hole 201 is equal to or larger than the diameter of the through-hole 201c, and it is, e.g., 2 - 3 µm or more.

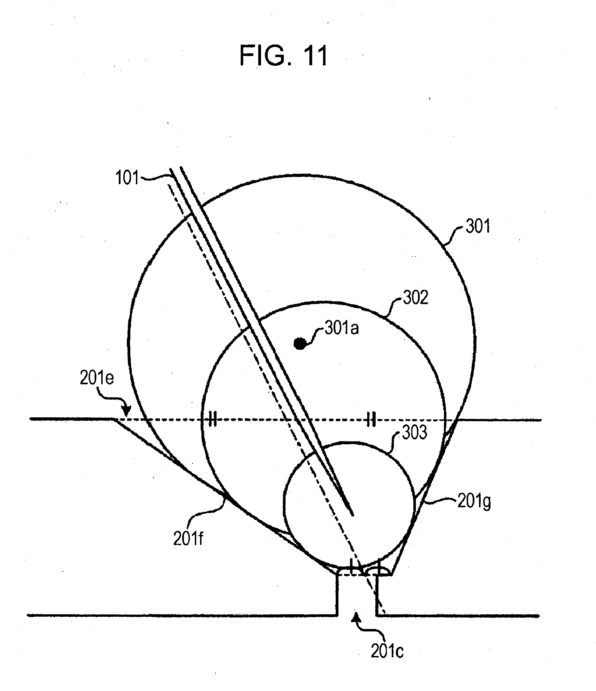

Fig. 11 is a sectional view showing a cross-section taken along a line XI-XI in Fig. 10. As shown in Fig. 10, a linear line (indicated by a one-dot-chain line in Fig. 11) connecting a center of the opening 201 e of the capturing hole 201 and a center of the bottom surface of the capturing hole 201 is substantially parallel to the longitudinal axis of the capillary needle 101. Further, the bottom surface of the capturing hole 201 is smaller than the opening 201 e and the entirety of the capturing hole 201 is formed in the shape of a funnel eccentrically tapering toward the through-hole 201 c. Stated another way, the inner surface 201f of the capturing hole 201 on the side nearer to the capillary needle 101 is moderately inclined relative to the horizontal direction, while the inner surface 201g of the capturing hole 201 on the side opposed to the capillary needle 101 is steeply inclined relative to the horizontal direction.

Thus, with this embodiment, since the capturing hole 201 is formed in the shape of an eccentric funnel, cells can be captured at respective positions within the capturing hole 201 corresponding to different cell sizes and cells having different diameters can be captured by one cell capturing plate 103. Also, with such an arrangement that the linear line connecting the center of the opening 201 e of the capturing hole 201 and the center of the bottom surface thereof is substantially parallel to the longitudinal axis of the capillary needle 101, when the capillary needle 101 is projected, the distal end of the capillary needle 101 reaches near the center of each of all the cells captured at respective positions within the capturing hole 201. As a result, the medical fluid can be reliably injected into each of the captured cells.

More specifically, as shown in Fig. 11, in the state that the large-sized cell 301, the medium-sized cell 302, and the small-sized cell 303 are captured in the capturing hole 201 at respective positions corresponding to different cell sizes, when the capillary needle 101 is projected, the distal end of the capillary needle 101 reaches near the center of each of the captured cells. In other words, when the stage adjusting mechanism section 107 and the capillary-needle moving mechanism section 108 are adjusted such that the distal end of the capillary needle 101 reaches near the center of the small-sized cell 303 captured near the through-hole 201 c of the capturing hole 201, the distal end of the capillary needle 101 passes near the center 301 a of the large-sized cell 301 captured near the opening 201 e of the capturing hole 201.

Therefore, the medical fluid delivered from the distal end of the capillary needle 101 can be reliably injected into the captured cell. Also, when the capillary needle 101 is projected, the distal end of the capillary needle 101 avoids contacting the end of the cell and pushing the captured cell away from the capturing hole. It is hence ensured that the distal end of the capillary needle 101 penetrates the cell membrane of the captured cell and the medical fluid is injected into the captured cell.

In the second embodiment, the cell capturing plate 103 is produced by employing the etching called RIE as in the first embodiment. In the second embodiment, however, since the inner surfaces 201f and 201g of the capturing hole 201 are continuously changed, the SOl substrate is inclined so as to continuously change the process conditions during the etching.

According to the second embodiment, as described above, the capturing hole for capturing the cell with suction via the through-hole bored in the cell capturing plate is formed in the shape of a gradually increasingly eccentric funnel tapering toward the through-hole, and the linear line connecting the centers of respective cross-sections of the capturing hole, as viewed perpendicularly to the direction in which the capturing hole is opened, is substantially parallel to the longitudinal axis of the capillary needle. Therefore, cells can be captured at respective positions suitable for the different cell diameters, and when the capillary needle is projected, the distal end of the capillary needle can reach near the center of each of the captured cells. Consequently, the cells having different diameters can be captured and the medical fluid can be reliably injected into each of the captured cells. In addition, since the cross-sectional area of the capturing hole is continuously changed, the diameters of the captured cells are not limited to discrete values.

While the cross-section of the capturing hole 201 as viewed perpendicularly to the direction in which the capturing hole 201 is opened is circular in each of the above-described embodiments, the cross-sectional shape is not limited to a circle, and it may be, e.g., elliptic or rectangular.

Further, as the material of the cell capturing plate 103, a quartz substrate or a glass substrate can also be used, by way of example, in addition to the SOl substrate.

Although a few embodiments have been shown and described, it would be appreciated by those skilled in the art that changes may be made in these embodiments without departing from the principles of the invention, the scope of which is defined in the claims and their equivalents.

## Claims

1. A cell capturing plate (103), comprising:
a flat surface (202);
a capturing hole (201) having an opening formed in a first side of the flat surface (202) in a direction substantially perpendicular to the flat surface (202) for supporting a cell, and a through-hole extending from the opening to a second, opposite side of the flat surface (202),
wherein an area of a first cross-section parallel to the flat surface (202) near the opening is larger than an area of a second cross-section parallel to the flat surface (202) near the through-hole and a linear line connecting centers of the first and second sides is oblique relative to the flat surface (202).

2. The cell capturing plate (103) according to Claim 1, wherein the capturing hole (201) has a shape formed by connecting a plurality of cylindrical portions having bottom surface areas which are gradually reduced toward the through-hole from the opening formed in the flat surface (202), and
wherein the through-hole extends from a bottom surface of one of the plurality of cylindrical portions having a minimum bottom surface area to the second side of the flat surface.

3. The cell capturing plate (103) according to Claim 1, wherein the capturing hole (201) has an upper-step cylindrical recess formed in continuation with the opening in the flat surface (202); and
a lower-step cylindrical recess opened to part of a bottom surface of the upper-step cylindrical recess, and
wherein the through-hole penetrates from a bottom surface of the lower-step cylindrical recess to the second side of the flat surface (202).

4. The cell capturing plate (103) according to Claim 3, wherein a linear line connecting a center of an opening of the upper-step cylindrical recess and a center of an opening of the lower-step cylindrical recess is oblique relative to the flat surface (202).

5. The cell capturing plate (103) according to any preceding claim, wherein the capturing hole (201) has a funnel-shaped portion in which a cross-section thereof parallel to the flat surface (202) has an area gradually reduced toward the through-hole from the opening in the flat surface (202), and
wherein the through-hole extends from a bottom area of the funnel shaped portion, which area is formed at a predetermined position in the funnel-shaped portion and extends parallel to the flat surface, to the second side of the flat surface (202).

6. The cell capturing plate (103) according to Claim 5, wherein a linear line connecting a center of the opening in the flat surface and a center of the bottom area of the funnel-shaped portion is oblique relative to the flat surface (202).

7. The cell capturing plate (103) according to any preceding claim, wherein the opening in the flat surface is formed in a circular shape with a diameter corresponding to 70 - 80% of a maximum diameter of the cell.

8. The cell capturing plate according to any preceding claim, wherein the through-hole is formed in a cylindrical shape having a bottom surface, the diameter of which is about 1 - 2 µm.

9. The cell capturing plate (103) according to any preceding claim, wherein the through-hole is formed in a cylindrical shape having a bottom surface, the diameter of which is about 10 - 20% of a minimum diameter of the cell.

10. A microinjection apparatus (100) having a capillary needle (101) being projectable along a longitudinal axis of the needle and delivering a material at predetermined timing for injection into a cell, and a cell capturing plate (103) having a flat surface (202) and capturing injection target cells, the cell capturing plate (103) comprising:
a capturing hole (201) having an opening formed in a first side of the flat surface (202) in a direction substantially perpendicular to the flat surface (202) for supporting the cell, and a through-hole extending from the opening to a second, opposite side of the flat surface (202),
wherein an area of a first cross-section parallel to the flat surface (202) near the opening is larger than an area of a second cross-section parallel to the flat surface (202) near the through-hole and a linear line connecting centers of the first and second cross-sections is substantially parallel to the longitudinal axis of the capillary needle (101).

11. The apparatus (100) according to Claim 10, wherein the capturing hole (201) has a shape formed by connecting a plurality of cylindrical portions having bottom surface areas which are gradually reduced toward the through-hole from the opening formed in the flat surface (202), and
wherein the through-hole extends from a bottom surface of one of the plurality of cylindrical portions having a minimum bottom surface area to the second side of the flat surface (202).

12. The apparatus (100) according to Claim 10, wherein the capturing hole has an upper-step cylindrical recess formed in continuation with the opening in the flat surface (202); and
a lower-step cylindrical recess opened to part of a bottom surface of the upper-step cylindrical recess, and
wherein the through-hole extends from a bottom surface of the lower-step cylindrical recess to the second side of the flat surface (202).

13. A method of producing a cell capturing plate (103) having a flat surface (202) and capturing a cell towards which a capillary needle (101) is projectable along a longitudinal axis of the needle, the method comprising the steps of:
forming, by etching, a through-hole (201) extending from a first side of the flat surface (202) to a second, opposite side of the flat surface;
forming, by etching, a first cylindrical portion which surrounds the through-hole (201) and which is located nearer to the second side of the flat surface (202); and
forming, by etching, a second cylindrical portion, which surrounds the first cylindrical portion,
wherein a linear line connecting a center of an opening formed in the first side of the flat surface (202) and a center of an opening of the first cylindrical portion, which is formed at a bottom area of the second cylindrical portion, is substantially parallel to the longitudinal axis of the capillary needle (101).
